# EUROPEAN PATENT SPECIFICATION

(11) **EP 2 308 983 B1**
(45) Date of publication and mention of the grant of the patent: **15.02.2012**
(21) Application number: 09012571.7
(22) Date of filing: 05.10.2009
(51) Int. Cl.: C12N 15/81, C12N 5/00, C07K 14/00

(54) **Cytochrome P450 from Rhizopus oryzae and uses thereof**
Cytochrom P450 aus Rhizopus oryzae und Verwendungen dafür
Cytochrome P450 à partir de Rhizopus oryzae et utilisations associées

(43) Date of publication of application: 13.04.2011
(73) Proprietor: Univerza v Ljubljani, 1000 Ljubljana (SI)
(72) Inventor: Bernhardt, Rita, 66129 Saarbrücken (DE); Cresnar, Bronislava, 1000 Ljubljana (SI); Hakki, Tarek, 66123 Saarbrücken (DE); Petric, Spela, 1000 Ljubljana (SI)
(74) Representative: Schmidt, Karsten

(56) References cited:
- US-A1- 2007 044 171
- MAKOVEC TOMAZ ET AL: "Catalytic and immunochemical properties of NADPH-cytochrome P450 reductase from fungus Rhizopus nigricans." JOURNAL OF STEROID BIOCHEMISTRY AND MOLECULAR BIOLOGY, vol. 82, no. 1, September 2002 (2002-09), pages 89-96, XP002570919 ISSN: 0960-0760
- KUNIC B ET AL: "Functional cloning, based on azole resistance in Saccharomyces cerevisiae, and characterization of Rhizopus nigricans redox carriers that are differentially involved in the P450-dependent response to progesterone stress" July 2001 (2001-07), MGG MOLECULAR GENETICS AND GENOMICS, VOL. 265, NR. 5, PAGE(S) 930-940 , XP002570920 ISSN: 1617-4615 * the whole document *
- DATABASE BIOSIS [Online] BIOSCIENCES INFORMATION SERVICE, PHILADELPHIA, PA, US September 2002 MAKOVEC TOMAZ ET AL: 'Catalytic and immunochemical properties of NADPH-cytochrome P450 reductase from fungus Rhizopus nigricans.' Database accession no. PREV200300047612 & JOURNAL OF STEROID BIOCHEMISTRY AND MOLECULAR BIOLOGY, vol. 82, no. 1, September 2002 (2002-09), pages 89-96, ISSN: 0960-0760

## Description

### Field of the invention

The present invention relates to a new cytochrome P450 from *Rhizopus oryzae* and to its use in the biotransformation of steroids. The present invention further relates to methods and materials for the hydroxylation of progesterone, testosterone, 11-deoxycorticosterone, and 11-deoxycortisol. The invention further relates to genes useful in the preparation of recombinant cells capable of biotransformation according to the invention, and to recombinant cells capable of biotransformation of steroids.

### Background Art

Hydroxylations of pharmaceutically important steroids, especially progesterone, carried out by filamentous fungi, are a well-studied class of bioconversions. The reactions are regio- and stereo-chemically specific and can replace several separate chemical steps in the synthesis of natural hormones and steroid drugs. The 11a-hydroxylations of progesterone performed by *Rhizopus* spp. or *Aspergillus* spp., and 11β-hydroxylations by *Curvularia* spp. or *Cunninghamella* spp. are currently established reactions used in the steroid industry mainly for the production of corticosteroids and progestogens.

Plenty of evidence indicates that cytochromes P450 (P450s or CYPs) are responsible for the hydroxylation of progesterone and some other steroids with similar skeleton structure in filamentous fungi, such as 7α- in *Phycommyces bakesleeanus,* 11β-*Cochliobolus lunatus,* 15α- in *Penicillium raistrickii* and 11α- in *Cunninghamella elegans, Nectria haematococca, Aspergillus fumigatus, Aspergillus ochraceus,* and *Rhizopus nigricans.* The P450 substrate specificity, the sites and stereochemistry of steroid oxygenation along with the range of hydroxylated derivatives depends on the substitution pattern of the substrate and the microorganism growth conditions. The steroid hydroxylation system in the fungi is a typical eukaryotic two-component system consisting of a NAD(P)H-cytochrome P450 reductase (CPR) and cytochrome P450 monooxygenase, which are anchored in the membrane of the endoplasmic reticulum, although cyt b₅ as an alternate electron donor can also be involved.

One of the most extensively studied fungal steroid hydroxylation systems is that of the zygomycete fungus *R. nigricans.* The microsomal enzyme activity is substrate inducible shortly after exposure of the fungus to steroids and is its main defense mechanism against toxic effects of steroids. So far, the fungal cytochrome P450 reductase (CPR1) has been purified, characterized and its involvement in fungal progesterone 11α-hydroxylation confirmed. The enzyme's cDNA was also functionally cloned and the inducibility of CPR proven. In contrast to CPR, the efforts to purify and/or clone the steroid monooxygenase of *R. nigricans,* or of any other filamentous fungi, have so far been unsuccessful.

Here we report the annotation of putative P450s from the sequenced genome of *R*. *oryzae* strain RA 99-880 and some characteristics of the fungal P450ome. The expression profiles of selected CYP genes during exposure of the fungus to progesterone were examined. Two of P450 genes [CYP509C10 (SEQ ID NO:2) and CYP509C12 (SEQ ID NO:1)] whose expression profiles best corresponded to the previously observed expression of CPR of *R. nigricans* and the 11α-progesterone production in both closely related fungi, were cloned and successfully expressed in *Schizosaccharomyces pombe.* The analyses of their progesterone hydroxylation capabilities demonstrated that the steroid was efficiently transformed to 11α- and 6β-hydroxylated derivatives by recombinant CYP509C12. In addition, we present evidence that the enzyme substrates were also testosterone, 11-deoxycorticosterone, and 11-deoxycortisol, as was observed in the fungus *R. oryzae.*

### Summary of the invention

The present invention relates to recombinant proteins and cells for the biotransformation of steroids. Methods and materials for biotransformation of steroids are also contemplated.

The present invention further relates to the use of a recombinant protein having the sequence set forth in SEQ ID NO:1, or a protein having a sequence which is at least 50%, 80%, 90%, 95%, 99%, 99.9% identical to SEQ ID NO:1 (CYP509C12), for the biotransformation of a steroid. In a preferred embodiment of the invention, the protein having a sequence which is at least 50%, 80%, 90%, 95%, 99%, 99.9% identical to SEQ ID NO:1 has the same catalytic function as has the protein of SEQ ID NO: 1.

In preferred embodiments of the invention said steroid is progesterone, testosterone, 11-deoxycorticosterone, and 11-deoxycortisol.

In another preferred embodiment of the invention, the biotransformation is a hydroxylation, more preferably a 11α hydroxylation and/or a 6β hydroxylation.

In another preferred embodiment of the invention, the use as described above is in combination with the use of a suitable recombinant cytochrome P450 reductase (CPR). In preferred embodiment of the invention, the CPR is RoCPR1 (SEQ ID NO:3).

The present invention also relates to the use of a gene coding for a recombinant protein having the sequence set forth in SEQ ID NO:1 for the production of a recombinant cell capable of biotransformation of steroids. The present invention also relates to the use of a gene coding for a recombinant protein having a sequence 50%, 80%, 90%, 95%, 99%, 99.9% identical to the sequence of SEQ ID NO:1, for the production of a recombinant cell capable of biotransformation of steroids.

The recombinant cell is preferably a *Schizosaccharomyces* cell, more preferably a *Schizosaccharomyces pombe* cell.

In preferred embodiments of the invention, the biotransformation is a hydroxylation, more preferably 11α hydroxylation and/or 6β hydroxylation.

In preferred embodiments of the invention, the recombinant cell further comprises a recombinant cytochrome P450 reductase, such as SEQ ID NO:3.

The invention further relates to a recombinant cell comprising a recombinant protein having the sequence set forth in SEQ ID NO:1, or comprising a recombinant protein having a sequence which is at least 50%, 80%, 90%, 95%, 99%, 99.9% identical to SEQ ID NO:1, said recombinant cell being capable of biotransformation of a steroid.

In preferred embodiments of the invention, said cell is capable of biotransformation of progesterone, testosterone, 11-deoxycorticosterone, and 11-deoxycortisol.

In preferred embodiments of the invention, said cell is capable of hydroxylation, more preferably of 11α hydroxylation and/or 6β hydroxylation.

Preferred recombinant cells of the invention are recombinant *Schizosaccharomyces* cells, more preferably *Schizosaccharomyces pombe* cells.

Preferred recombinant cells of the invention further comprise a recombinant cytochrome P450 reductase, such as (SEQ ID NO:3).

Preferred recombinant cells of the invention are capable of hydroxylation of a steroid, more preferably capable of 11α hydroxylation and/or 6β hydroxylation of a steroid, most preferably of 11a hydroxylation and/or 6β hydroxylation of progesterone, testosterone, 11-deoxycorticosterone, and 11-deoxycortisol.

11α hydroxylation of progesterone is most preferred.

The present invention also relates to the use of recombinant cells of the invention for the biotransformation of a steroid. In preferred embodiments of the invention, the biotransformation is hydroxylation of progesterone, testosterone, 11-deoxycorticosterone, and 11-deoxycortisol. Most preferable, the hydroxylation is 11α hydroxylation and/or 6β hydroxylation.

The present invention also relates to a method for the production of a product steroid from a substrate steroid, said method comprising (a) incubating said substrate steroid with a recombinant cell of any of claims 5 to 8 in a suitable medium, whereby said substrate steroid is converted to said product steroid, and (b) obtaining said product steroid from the medium. Said conversion is preferably in a fermentation process.

Preferably, the substrate steroid is selected from the group consisting of progesterone, testosterone, 11-deoxycorticosterone, and 11-deoxycortisol.

Preferably, the substrate steroid is hydroxylated, more preferably 11α hydroxylated and/or 6β hydroxylated.

11α hydroxylation of progesterone is most preferred.

In preferred embodiments of the invention, the product steroid is 11α-progesterone.

### Brief description of the figures

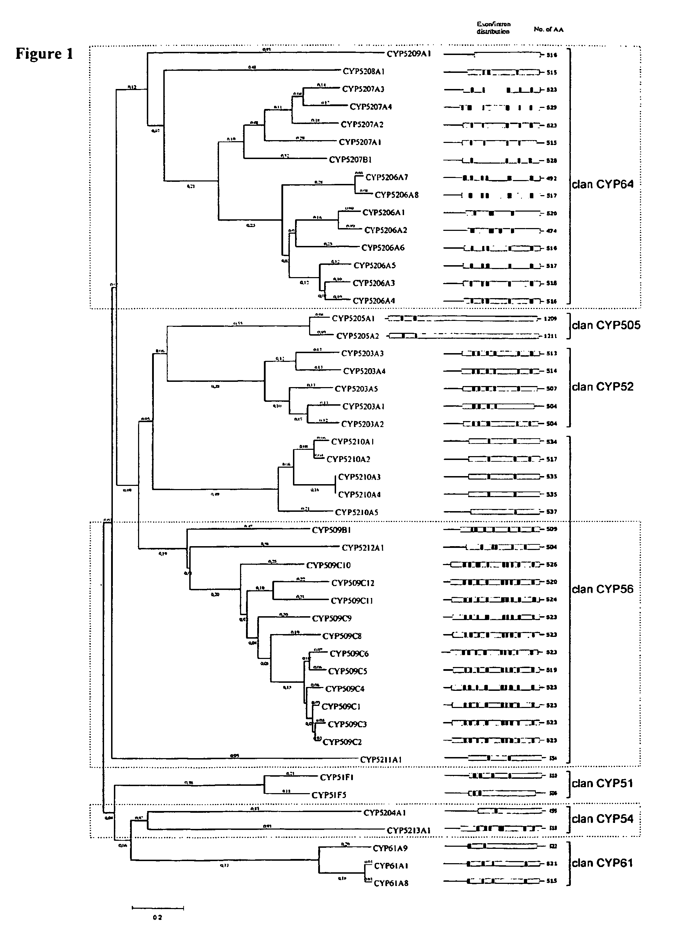

Figure 1: Phylogenetic tree of putative cytochromes P450s from *R*. *oryzae.* The bootstrapped (N=1000) neighbor-joining tree was compiled using MEGA version 3.1.
Clans are marked by brackets and the fungal genes analyzed for expression in the presence of progesterone are presented in grey boxes.

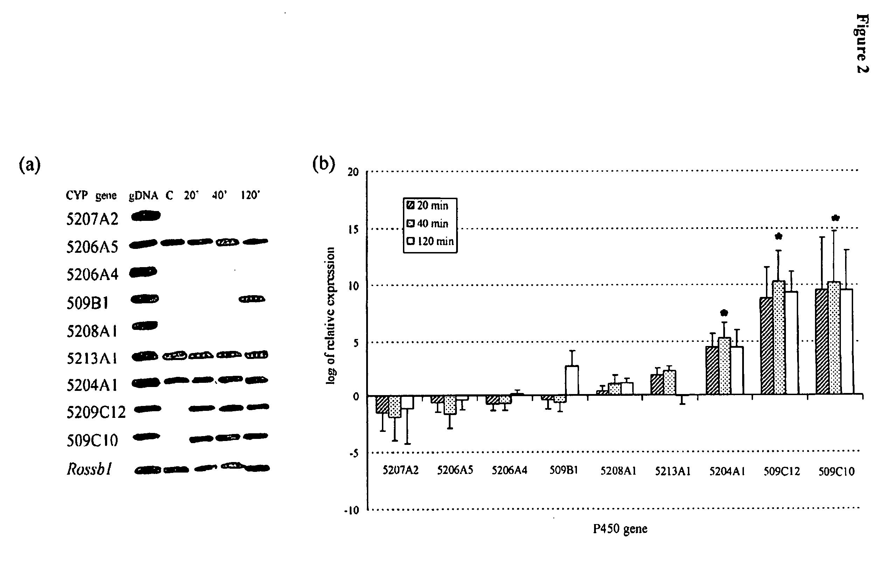

Figure 2: Expression profiles of selected *R. oryzae* putative P450 genes. (a) RT PCR for determining P450 transcripts in the fungal control cultures and cultures treated with progesterone for indicated time periods. Housekeeping *Rossb1* gene of fungus *R*. *oryzae* was used as a reference for the amount of starting templates in PCR reactions. (b) The relative amounts of P450 mRNAs determined by real time RT PCR using the same cDNA templates as above and the *Rossb1* transcript levels as the endogenous reference. Error bars represent standard deviation of 6 independent experiments. The asterisks indicate a significant difference in expression of P450s compared to the control. Abbreviations: C, fungal cultures not exposed to progesterone, gDNA, genomic DNA and *Rossb1* gene, gene encoding *S*. *cerevisiae* ribosome-associated Hsp70 (SSB1) homologue of *R. oryzae.*

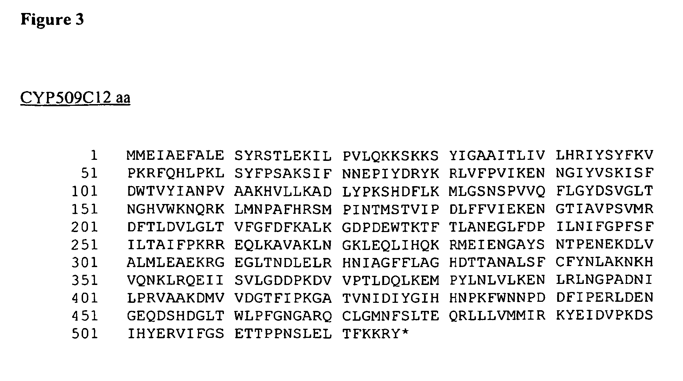

Figure 3: Amino acid sequence of CYP509C12.

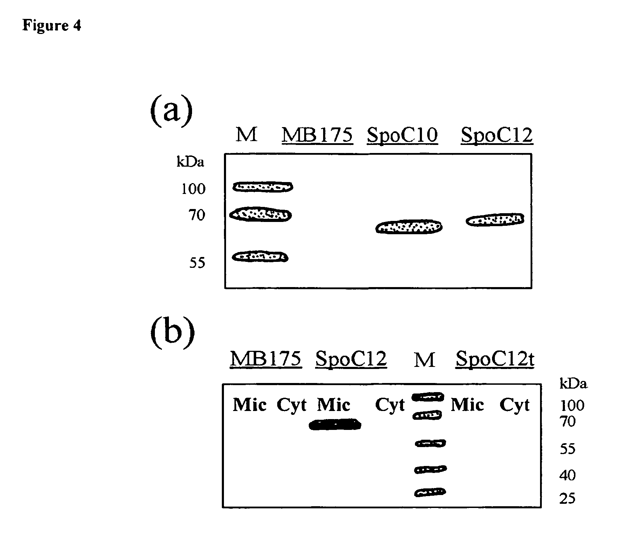

Figure 4: Heterologous expression of *R*. *oryzae* CYP509C12 and CYP509C10 genes. (a) Western blot analysis performed on whole cell lysate protein samples (30µg) of parental fission yeast strain and strains SpoC10 and SpoC12 expressing *R. oryzae* CYP509C12 and CYP509C10 genes, respectively. (b) The same analysis carried out on sub-cellular protein samples (5µg) of parental fission yeast strain and strain SpoC12 in the absence and presence of plasmid promoter repressor (thiamine). The proteins were separated by SDS PAGE, transferred to the membrane and the recombinant proteins were detected using His₆ tag monoclonal antibody as described in Experimental Procedures. Abbreviations: MB 175, parental S. *pombe* strain, Cyt, cytosolic fraction, Mic, microsomal fraction, M, protein standard and t, thiamine.

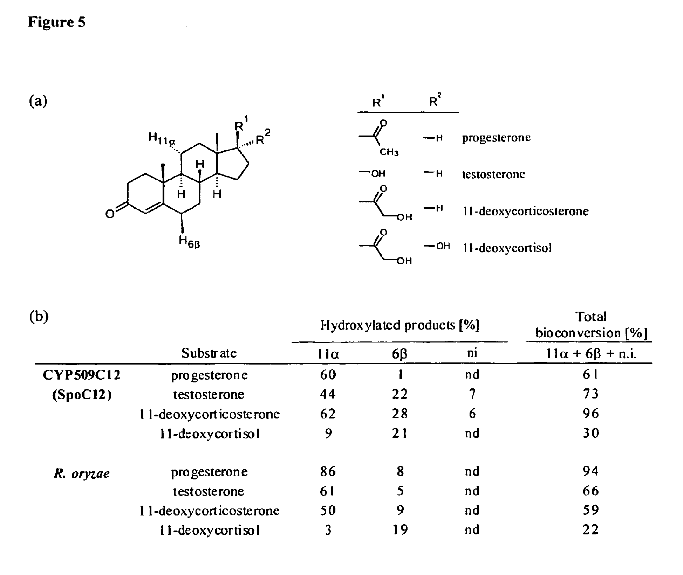

Figure 5: Steroid hydroxylation activity in *S*. *pombe* strain SpoC12 expressing CYP509C12 and fungus *R. oryzae.* (a) General structure of steroids used in this study. (b) The assays of hydroxysteroid derivatives production were performed as described in Experimental Procedures and the steroid samples were analyzed by UPLC/MS. Data shown are mean values of four independent experiments. Abbreviations: *nd*, not detected and *ni,* not identified monohydroxylated steroids.

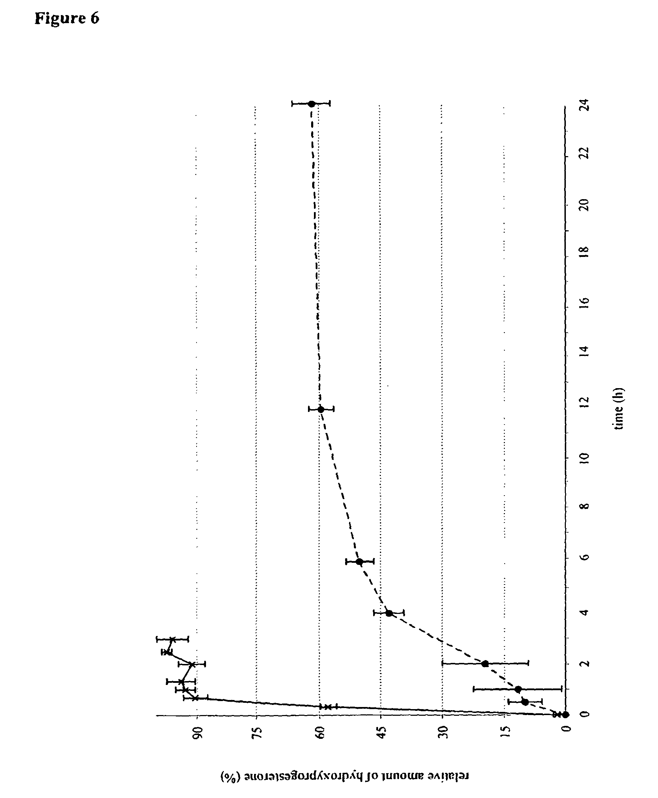

Figure 6: Temporal analysis of hydroxyprogesterone production in S. *pombe* strain Spo12 expressing recombinant CYP509C12 and in fungus *R. oryzae.* The assays were done as described in Experimental Procedures. Values are means of six replicates and error bars indicate standard deviation.

### Detailed description of the invention

Within the context of the present invention, the "% identity" of a protein relative to a reference protein of a defined length shall be understood as follows: A peptide that is 50 percent identical to a reference polypeptide that is 100 amino acids long can be a 50 amino acid polypeptide that is completely identical to a 50 amino acid long portion of the reference polypeptide. It might also be a 100 amino acid long polypeptide, which is 50 percent identical to the reference polypeptide over its entire length. Of course, other polypeptides will meet the same criteria. The term "sequence identity" as used herein means that the sequences are compared as follows. The sequences are aligned using Version 9 of the Genetic Computing Group's GAP (global alignment program), using the default (BLOSUM62) matrix (values -4 to +11) with a gap open penalty of -12 (for the first null of a gap) and a gap extension penalty of -4 (per each additional consecutive null in the gap). After alignment, percentage identity is calculated by expressing the number of matches as a percentage of the number of amino acids in the reference polypeptide.

Within the context of the present invention, the term "biotransformation" shall be understood to mean the chemical modification (or modifications) made by an organism on a chemical compound. In a preferred embodiment, biotransformation means the chemical alteration of chemicals such as nutrients, amino acids, toxins, or drugs by the organism. In preferred embodiments of the invention, biotransformation is performed by a single cell organism.

Preferred biotransformations of the present invention are oxidation and reduction by monooxygenases, such a cytochrome P 450.

"Hydroxylation", within the context of the present invention, shall be understood to be any chemical process that introduces one or more hydroxyl groups (-OH) into a compound, thereby oxidizing the same.

A "medium", within the context of the present invention, shall be understood to be any medium which supports survival of the recombinant organism, and permits the bioconversion reactions of the current invention. Suitable media preferably contain nutrients and water. They can be liquid media or solid media, such as agar agar.

A "recombinant cell", within the context of the present invention, shall be understood to be a living cell which comprises at least one recombinant gene. A recombinant gene shall be understood to be a gene which is introduced into said cell by recombinant DNA technology or other non-natural processes. A recombinant gene can also be a gene which is naturally occurring in the cell, but the expression level of which is artificially increased by recombinant DNA technology. A recombinant cell can thus be a cell comprising a recombinant heterologous gene, or a homologous gene which is recombinantly over-expressed.

A "recombinant polypeptide", within the context of the present invention, shall be understood to be polypeptide which is introduced into said cell by recombinant DNA technology or another non-natural process. A recombinant polypeptide can also be a polypeptide which is naturally occurring in the cell, but the expression level of which is artificially increased by recombinant DNA technology.

The present invention relates to recombinant polypeptides and cells for the biotransformation of steroids.

In an attempt to isolate the progesterone-inducible cytochrome P450(s) responsible for the steroid 11α-hydroxylation by the zygomycete fungus *Rhizopus oryzae, 48* putative P450 genes were manually annotated from the fungal genome. Structural and phylogenetic analysis showed that these P450 genes of the fungus could be classified into 14 families including four subfamilies and clustered under 7 fungal P450 clans. Two of the P450 families were highly conserved, whereas the others represented novel fungal P450 families with unknown functions. The examination of induction of selected P450 genes by progesterone revealed that 5 genes were up-regulated shortly after exposure of *R. oryzae* to this steroid, albeit to varying extent. Two highly induced genes, CYP509C12 and CYP509C10, were expressed in *Schizosaccharomyces pombe.* Their functional analyses demonstrated that recombinant CYP509C12 was capable of hydroxylating progesterone as well as testosterone, 11-deoxycorticosterone, and 11-deoxycortisol, while the steroid hydroxylation activity of recombinant CYP509C10 had not been proven. The substrates of CYP509C12 were hydroxylated predominantly at 11α and 6β positions of steroid ring system, similarly to what was shown in *R. oryzae* cultures exposed to the steroids.

The steroid biotransformation activities of parental *S. pombe,* SpoC10 and SpoC12 were tested after cultivation of the yeast for 24 h in the presence of progesterone and the absence of thiamine. HPLC analyses of the culture steroid extracts showed that all of them were hydroxylated by SpoC12, while the reaction was not accomplished neither by strain SpoC10 nor the parental strain (data not shown). Therefore, further studies of hydroxylation efficiency and hydroxylated derivatives identification were performed only on the CYP509C12 expressing strain. The extracted steroids were analyzed by LC/MS. Figure 5 shows that SpoC12 transformed progesterone, testosterone, DOC, and RSS. The efficiency of total steroid hydroxylation, the distribution between 11α- and 6β-hydroxylated derivatives and the appearance of non-identified monohydroxysteroids depended on substrate. The most efficiently transformed was DOC (96 %) and to a lesser extent testosterone (73 %) and progesterone (61 %). The hydroxylation of RSS was least efficient (30 %). The hydroxylation of progesterone was carried out mainly at 11α position of steroid ring system (60 %), whereas the same proportion between 11α- and 6β-hydroxylated products (2:1) was observed with testosterone and DOC as substrates. Among steroids used in our study, RSS represented an exception regarding the distribution between 11α- and 6β-hydroxylated derivatives, since more than half of total hydroxylated products represented 6β-RSS. In contrast to progesterone and RSS, which were transformed only into 11α- and 6β-hydroxylated derivatives, the hydroxylation of testosterone and DOC also resulted in production of non-identified monohydroxysteroids.
In order to compare fission yeast strain SpoC12 and *R. oryzae* steroid biotransformation capability, studies on fungal hydroxylation were performed. After hydroxylation activity assays, the steroids were extracted and analyzed by LC/MS. Beside progesterone, a known substrate of *R. oryzae* monooxygenase(s), other steroids were also transformed to 11α- and 6β- hydroxylated products, albeit to different extent (Figure 5). In addition, three discrepancies appeared between transformation activities of the fungus compared to recombinant yeast: (i) the fungus hydroxylated DOC to a much lesser extent than SpoC12; (ii) the ratio between 11α-testosterone and 6β-testosterone in the fungus was much higher as in SpoC12; (iii) finally, all steroids were hydroxylated only at 11α- and 6β- positions of the steroid ring system in *R. oryzae,* whereas other monohydroxysteroid products were also detected in the recombinant yeast.

With the aim to determine the relative rate of progesterone hydroxylation by fission yeast strain SpoC12 and by the *R. oryzae* mycelium, time dependant accumulation of the hydroxylated steroids was followed. The relative conversion rate of progesterone to monohydroxylated products estimated by linear regression was determined to be 0.091 µmol h⁻¹ (R² = 0.982) for strain SpoC12, while this rate was 1.190 µmol h⁻¹ (R² = 0.971) for the *R. oryzae* cultures (Figure 6).

The sequenced genome of the filamentous zygomycete fungus *Rhizopus oryzae* strain RA 99-880 enabled us to identify 48 putative cytochrome P450 genes (Figure 1). The number of CYP genes in genomes of different fungi ranges from two in fission yeast *S. pombe* to several tens in filamentous fungi, as in ascomycetes *Aspergillus* and *Fusarium* spp. and the basidiomycete white-rot fungus *Phanerochaetae chrysosporium,* which has the largest fungal P450ome to date (149 genes). In general, the genomes of saprophytic and pathogenic filamentous fungi contain a large number of P450 genes. So far, with the exception of CYPs with a conserved role in endogenous metabolism (such as CYP51 and CYP61), the functions of few fungal P450s have been proven. Amongst these are some P450s involved in secondary metabolite biosyntheses in *Aspergillus* and *Fusarium* spp. and in xenobiotic degradation and/or detoxification in *Aspergillus* spp. and *P. chrysosporium.*

The putative CYPs of *R. oryzae* were grouped into 12 novel and two existing fungal families (CYP51 and CYP61) (Figure 1). A similar number of families was found in basidiomycete fungi, while P450 genes within filamentous ascomycete fungi, with the exception of *Neurospora crassa,* fall into more than 70 distinct families. The average number of *R. oryzae* genes per family (approx. 3 genes) is close to the number found in ascomycete fungi (1 - 2 genes), but considerably less than those found in the filamentous basidiomycete *P. chrysosporium* (12 genes).

Clan-based classification *of R. oryzae* P450 families showed that 13 of them were grouped into 7 previously identified fungal clans and one family (CYP5209A1) could only be related to clan CYP64 (Figure 1). The number of families assigned to a particular clan ranged from one to four. Two of them, clan CYP56 and clan CYP64, both containing 4 CYP families with a total of 19 and 15 members, respectively, were extensively diversified in *R. oryzae.* Clan CYP64 also has several members in P. *chrysosporium* (54 genes). On the other hand, clan CYP56 is sparsely represented in genomes of sequenced basidiomycete and ascomycete fungi, including those *Aspergillus* species, which are known to hydroxylate steroids. The diversification of clan CYP56 therefore seems to be specific for the zygomycete fungi. Since this clan also contains the steroid hydroxylase CYP509C12, it implies the origin of steroid detoxifying P450s in Zygomycete and Ascomycete fungi is polyphyletic.

RT PCR and real time RT PCR analysis of expression profiles of selected *R. oryzae* P450 genes during exposure of the fungus to progesterone demonstrated that the genes were differentially expressed under tested conditions (Figure 2). The results suggested that metabolic pathways involving putative CYPs could either decline or increase during steroid stress. Similarly, P450 gene expression studies in *Arabidobsis* (Narusaka et al. 2004) and *P. chrysosporium* (Doddapaneni & Yadav 2005;Makovec & Breskvar 2002;Yadav & Doddapaneni 2003;Yadav et al. 2006) showed that the response of a specific P450 to various stresses was strictly regulated and connected to its function. Expression profile analyses of P450s assigned to primary metabolism clans CYP505 and CYP61 in the related fungus *R. nigricans* showed that these fungal monooxygenases were up-regulated in response to progesterone (unpublished data). Thus, besides observing up-regulation, the time-course profiles and mRNA abundance was crucial for distinguishing between steroid induced hydroxylases and P450s involved in other metabolic functions. High levels of CYP509C12 and CYP509C10 transcripts were present in *R. oryzae* only after exposure to progesterone, similarly to what has been shown for the *R. nigricans* CPR involved in the steroid hydroxylation system (Makovec & Breskvar 2002; Kunic et al. 2001). We therefore assumed these two genes could encode steroid hydroxylating enzymes.

The expression *of R. oryzae* CYP509C10 and CYP509C12 genes in *S*. *pombe* strains, designated SpoC10 and SpoC12, respectively, was confirmed (Figure 4), but only strain SpoC12 efficiently transformed the tested steroids (Figure 5). These findings suggested that the recombinant CYP509C10 was either not properly assembled, which hindered the acceptance of reducing equivalents from redox partners and/or substrate binding, or the enzyme had a role in some other process affected by progesterone.

The LC/MS analyses of steroids extracted from SpoC12 cultures demonstrated that the recombinant enzyme transformed the tested steroids (progesterone, testosterone, DOC and RSS) into 11α-and 6β-hydroxylated derivatives and some non-identified monohydroxysteroids, when testosterone and DOC were used as substrates (Figure 5). It is therefore evident that the fission yeast's NAD(P)H-cytochrome P450 reductase was a suitable redox partner of CYP509C12 steroid hydroxylase, although involvement of cytochrome b₅ and its reductase cannot be excluded (reviewed in Hannemann et al. 2007). The slight differences between the steroid hydroxylation activities of strain SpoC12 in comparison to *R. oryzae* cultures could be the result of different growing conditions of the microorganisms.

A 10-fold slower rate of progesterone hydroxylation in recombinant fission yeast compared to *R. oryzae* cultures was determined (Figure 6). Thus the efficiency of interaction between enzymes of different species was suboptimal and/or the amount of fission yeast CPR was inadequate to supply electrons to the over-expressed *R. oryzae* CYP509C12. Studies *of R. nigricans* steroid hydroxylation system have shown both components are induced in response to progesterone, maintaining the appropriate ratio between both partners. Co-expression of both fungal enzymes increases the rate of steroid hydroxylation, as was previously observed in fission yeast co-expressing human CYP11B1 and the corresponding electron transport proteins (Hakki et al. 2008).

### Examples

### Example 1: Strains, Culture Growth Conditions, and Chemicals

The filamentous fungus *R. oryzae* strain RA 99-880, obtained from the FGSC (Fungal Genetics Stock Center, University of Missouri, Kansas City, USA), was cultivated for 18 h at 28 °C as described previously (Breskvar & Hudnik-Plevnik 1977). Where required, the mycelium was exposed to 300 µM final concentration of steroids progesterone or 11-deoxycorticosterone dissolved in DMFO for up to 2 h.

Cultivation of fission yeast *Schizosaccharomyces pombe* has been summarized in (Moreno et al. 1991) and (Alfa et al. 1993). Briefly, parental fission yeast strain MB175 (genotype *h⁻ade6.M210 ura4*.d118 *leu1.32 his3*.Δ1) was cultivated at 28 °C, 180 rpm in Edinburgh minimal medium (EMM) supplemented with leucine, histidine, uracil, and adenine. 10 mL liquid cultures were grown over night and used for the inoculation of main cultures. After approximately 18 hours of growth, log phase cells were harvested and cell densities determined using a haemocytometer. All subsequent experiments were performed with cultures whose cell densities reached the range of 10⁷ - 5 × 10⁷ cells mL⁻¹.

Steroids progesterone, testosterone, 11-deoxycorticosterone (DOC), and 11-deoxycortisol (RSS) used in transformation studies were purchased from Sigma, USA. 11α- and 6β-derivatives of progesterone, testosterone, DOC, and RSS were obtained from Steraloids (Newport, RI, USA). All other chemicals and solvents were analytical grade.

### Example 2: Putative P450ome Annotation

The genome sequence of *R. oryzae* (assembly 3) was downloaded from the Broad Institute Database (http://www.broad.mit.edu/annotation/genome/rhizopus_oryzae/ MultiHome.html). To account for high variability of the primary sequences of CYPs, screening for putative *R. oryzae* P450 genes involved comparing its genomic sequence to a collection of 956 CYP amino acid sequences from various organisms using blastall 2.2.12 (tblastn 2.2.12). These included 110 *Fusarium graminearum,* 289 *Arabidopsis thaliana,* 126 human, and 43 bacterial cytochromes P450, all obtained from Dr. Nelson's Cytochrome P450 homepage (http://drnelson.utmem.edu/CytochromeP450.html). With the E value set at 10, 29473 hits were generated and visualized on the *R. oryzae* genomic map, revealing 50 potential CYP loci. Further, a process of ORF identification was carried out on each locus, starting from the conserved heme-binding domain (GxxxCxG) and stretching in both directions. Intron positions were predicted according to the conserved GT/AT splice junction sequence. The predicted 5' and 3' ends of genes, signified by Met or the stop codon, respectively, were verified by aligning them to CYPs deposited in GenBank. To improve the quality of the putative sequences, the process was repeated several times. The intron-exon boundaries as well as structural compliance to fungal clans were used to check the accuracy of our final alignments.

### Example 3: Alignment Analysis and Phylogenetic Tree Construction

The 48 *R. oryzae* deduced P450 protein sequences were aligned with Vector NTI Advance 9.1.0 (Invitrogen) and grouped into families according to 40 % sequence identity. The BLAST algorithm (NCBI) was used for comparison of deduced P450 proteins to P450 sequences originating from other organisms. Phylogenetic analyses and tree construction were conducted using MEGA version 3.1 (Kumar 2004), generating bootstrapped (N=1000) neighbor-joining trees. Finally, the predicted P450 genes were submitted to Dr. Nelson for confirmation, naming, and clan designation (Nelson 1999).

### Example 4: RT PCR and Real Time RT PCR

RNA was isolated from the fungus treated with progesterone for 20, 40 or 120 min as well as from the non-treated mycelium as described previously (Breskvar et al. 1991). Samples of 2 µg of RNA were treated with DNAse 1 and reverse-transcribed with random hexamer primers using RevertAid™ H Minus First Strand cDNA Synthesis Kit (Fermentas, Canada) following the manufacturer's instructions. Based on multiple alignments of deduced P450 families, the amplification of the cDNA of 31 P450 genes was carried out under conventional conditions using gene-specific primers which were designed with Primer Express (Applied Biosystems, CA, USA, (AB)). The specificity of each primer pair was verified by sequencing of amplified genomic fragments (Sequiserve, Vaterstetten, Germany).

Quantitative RT PCR using SYBR green Mastermix (AB) was performed for 9 selected genes in an ABI Prism 7500 Real Time System (AB) according to the manufacturer's instructions. Following amplification, a dissociation curve was performed on all PCR products to additionally ensure their specificity. The relative quantity of cDNA was calculated with the standard curve method and calibrated with a *R. oryzae* housekeeping *Rossb1* gene (Broad ref. RO3G_01864.1), a homologue of the *Saccharomyces cerevisiae* ribosome-associated Hsp70 (SSB1), whose expression is not affected by steroid treatment. The expression levels of each P450 gene were normalized to those in the untreated control. The statistical significance of up- or down-regulation was evaluated using Wilcoxon's test with a cutoff p-value < 0.05.

### Example 5: Construction ofR. oryzae cDNA Library and P450 cDNA Amplification

Poly(A)-RNA was prepared from total RNA of fungal mycelia previously exposed to 300 µM progesterone for 20 min by oligo(dT) column (Stratagene) and cDNA was synthesized using a ZAP Express cDNA Synthesis kit (Stratagene). The library was constructed in the ZAP Express™ expression vector (Stratagene). All procedures were performed according to the instructions of manufacturer. Full-length cDNAs of two fungal monooxygenases (CYP509C10 and CYP509C12) were amplified from 1 µL of cDNA library under conventional conditions using primers listed in Figure 7.

### Example 6: Expression Vector Preparation and Transformation of Fission Yeast

Amplified cDNAs of CYP509C10 and CYP509C12 lacking the terminal stop codon were cloned into pNMT1-TOPO (Invitrogen) His₆ tag expression vector. After transformation of the *E*. *coli* Top10 strain, the cDNA sequences were determined. Recombinant expression vectors were introduced into fission yeast by the lithium acetate method (Ito 1983). The selection of recombinant yeast was performed through the *Leu2 Saccharomyces cerevisiae* gene carried on the pNMT1-TOPO vector that functionally complements the *leu1* mutant *S. pombe* recipient strain (Andreadis et al. 1984). During transformation the NMT1 promoter was repressed by 5 µM of thiamine. The obtained fission yeast strains were designated as SpoC10 and SpoC12, respectively. The CYP509C10 and CYP509C12 gene expression, was achieved by cultivating fission yeast in the absence of thiamine as described previously (Moreno et al. 1991).

### Example 7: Preparation of Whole Cell Lysate and Subcellular Fraction Proteins

*S. pombe* parental strain, SpoC10, and SpoC12 cultures were grown as described in the previous sections. For whole cell lysate preparation 10⁹ cells were washed twice with 50 mL of water (4000 x g, 5 min) and suspended in 500 µL of protein extraction buffer (20 mM Tris, pH 7.5, 5 mM MgCl₂, 2 mM EDTA, 1 mM DTT, 1 mM PMSF, 0.1 % Triton x-100) containing proteinase inhibitors (10 µg mL⁻¹ antipaine, pepstatine, leupeptine, chimotrypsine), then disrupted by vigorous shaking with glass beads for 3 min. The suspension was centrifuged at 13500 x g for 10 min and the supernatant was used for subsequent experiments.

Subcellular fractions were prepared by a method modified from that of (Oeda et al. 1985). 2 x 10⁹ cells of *S. pombe* parental strain and SpoC12 were washed with water, suspended in 200 µl of buffer B1 (100 mM Tris-H₂SO₄, pH = 9.4, 1.2 M sorbitol) and incubated at room temperature for 10 min. Following centrifugation (4000 x g, 10 min) the cell pellet was suspended in 1 mL of buffer B2 (KH₂PO₄, pH=7.4, 1.2 M sorbitol) and incubated at 30 °C with 20 mg of Zymolyase (ICN Biomedicals, Aurora, OH, USA). The sphaeroblasts were centrifuged (4000 x g, 5 min) and suspended in protein extraction buffer. Cell breakage was carried out with an MSE sonicator (MSE Scientific Instruments, UK) using an intensity setting of 9 µm amplitude. To remove cell debris the homogenate was centrifuged at 3000 x g for 5 min and the obtained supernatant at 10000 x g for 20 min. The microsomal and cytosolic fractions were separated by centrifugation of the supernatant at 125000 x g for 90 min. Sediments containing the microsomal fraction were suspended in buffer A (10 mM Tris-HCl, pH=7.5, 0.65 M sorbitol). During the procedures the samples were kept on ice and the centrifugations were done at 4 °C. Protein concentrations were determined by the Bradford method (Bradford 1976) with Nanoquant reagent (Roth) and BSA as a standard.

### Example 8: Western Blot Analysis

30 µg of whole cell lysate proteins or 5 µg of cytosolic or microsomal fraction proteins were separated by 10 % SDS polyacrylamide gel electrophoresis (Laemmli 1970) and electroblotted onto the Hybond⁺ nitrocellulose membranes (Amersham Bioscience, USA). Immunodetection was performed using antibodies against the hexahistidine tag (1:1000 dilution) and secondary antibodies conjugated with HRP (1:20,000 dilution) (Santa Cruz Biotechnology) by the SuperSignal West Pico detection system (Thermo Scientific Pierce) according to manufacturer's instructions.

### Example 9: Steroid Hydroxylation Assays of Recombinant S. pombe and R. oryzae

*S. pombe* cells, grown as described in the previous section, were washed with EMM, centrifuged (4000 × g, 5 min, 4 °C) and suspended in 10 mL of fresh medium to a final density of 10⁸ cells mL⁻¹. Steroids progesterone, testosterone, DOC, and RSS were added to the final concentration of 300 µM and the cultures were incubated at 28 °C with shaking for up to 24 h. 500 µL of SpoC12 yeast culture was taken after different time periods and the steroids were extracted twice with chloroform, evaporated, solved in acetonitrile, and analyzed by HPLC. The steroid extracts from whole cell cultures growing for 24 h were analyzed by LC/MS.

The *R. oryzae* hydroxylation activity was determined as described previously (Hudnik-Plevnik & Cresnar 1990). Briefly, 1 g of moist DOC treated fungi, grown as described in the previous section, was washed with saline and suspended in 10 mL of solution A (1 mM Na₃PO₄, 0.2 mM EDTA dinatrium salt, and 0.04 mM glutathione, pH 5.5). Cycloheximide and different steroids (progesterone, DOC, RSS and testosterone) were added to the final concentration of 300 µM. The mycelia were incubated at 28 °C with shaking for up to 2 h. The collections of fungal cultures and the analyses of steroid extracts were done as above.

### Example 10: HPLC and UPLC/Mass Spectrometry Analysis

Reverse phase HPLC using an isocratic solvent system of acetonitrile:H₂O 60:40 (v/v) as mobile phase, flow rate 1 mL⁻¹ min, was carried out on a WellChrom system (Knauer, Berlin, Germany) composed of an auto-sampler AS-2050 plus, Mini-Star K-500 pump, Jet Stream II Plus thermostat, and an UV-detector WellChrom Spectro Photometer K-2600, equipped with a C18 ODS Hypersil 250 x 4.6 mm, 5 µm particle column from Thermo^{®} (Waltham, MA, USA). Steroids were detected at 240 nm and the peak area calculation was done using the EuroChrom 2000 software (Knauer). Steroid standards were used to identify the HPLC peaks and to construct calibration curves. Concentrations of steroids were determined using calibration curves in the range of 0.05 -1 mg mL⁻¹ of each analyte. Similar absorption coefficients of substrates and their respective metabolites were verified by producing standard curves for each steroid and products. The peak area of a specific steroid at each time point was assessed relative to the total area of identified steroid peaks.

Mass measurements were run on a hybrid quadruple time of flight mass spectrometer (Q-TOF) provided with an orthogonal Z-spray ESI interface (Waters Micromass, Manchester, UK). Mass spectrometer was interfaced to an ultra performance liquid chromatography (UPLC) system based on a Waters Acquity (Waters, Milford, USA) binary pump with a BEH C-18 column (1.7 µm, 50 mm x 2.1 mm i.d.). The mobile phases consisted of water and acetonitrile with mixture of 0.1 % of formic acid in water. Compressed nitrogen (99.999 %, Messer, Slovenia) was used as both the drying and the nebulizing gas. The nebulizer gas flow rate was set to approximately 20 L h⁻¹ and the desolvation gas flow rate to 600 L h⁻¹. A cone voltage of 30 V and a capillary voltage of 2.7 kV were used in positive ion mode. The desolvation temperature was set to 250 °C and the source temperature to 150 °C. The mass resolution of approximately 9500 was used for determination of elemental composition with TOF mass spectrometer. Full width of the peak was measured at half of its maximum height. MS were acquired in centroid mode over an *m*/*z* range of 50-1000 in scan time 0.25 s and inter scan time 0.05 s. The data station operating software was Mass Lynx v 4.1 (Micromass, Manchester).

### Example 11. R. oryzae P450ome Analysis

From the P450 signature sequence-containing loci identified in the genome of R. *oryzae,* we manually annotated 48 putative CYP genes and two pseudogenes, and the latter were omitted from further analysis. In all but one P450 gene the reading frames were interrupted by one to nine introns. The lengths of encoded enzymes ranged from 470 to 1211 amino acids. The phylogenetic analysis using the primary amino acid sequence identity criterion revealed that *R. oryzae* P450s group into 14 CYP families. Eight of the P450 families have more then one member and the P450s of two of them are divided into two subfamilies (Supplemental file 2). Among *R. oryzae* P450 families CYP51 (2 members) and CYP61 (3 members) families represented highly conserved sterol 14-α demethylases and sterol C22 desaturases, respectively, involved in the cell wall ergosterol biosynthesis pathway. The remaining 12 families were classified as novel fungal P450 families and grouped into 7 clusters of cross-species P450 families, i.e. clans, by the P450 nomenclature committee (c/o Dr. D. R. Nelson, University of Tennessee, Memphis) (Figure 1).

One *R. oryzae* P450 family containing 5 members is related to clan CYP52. The proteins of CYP52 family were first identified in the *Candida* species (Sanglard & Loper 1989) as enzymes associated with the terminal hydroxylation of n-alkanes and ω-hydroxylation of fatty acids. Four *R. oryzae* families, of which family CYP509 was distinctly diversified and included 14 members, were clustered under clan CYP56. Originally CYP56 (also known as Dit2) was identified in *Saccharomyces cerevisiae* as an enzyme involved in the biosynthesis of N,N'-bisformyl dityrosine, a component of the outer layer of the spore cell wall (Briza et al. 1996). Two new P450s of clan CYP505 were also identified in the fungus' genome. The original member of this clan, *Fusarium oxysporum* CYP505, also known as P450foxy, catalyzing fatty acid subterminal hydroxylation (Kitazume et al. 2000), was characterized as the eukaryotic counterpart of bacterium *Bacillus megaterium* CYP102 (Boddupalli et al. 1992). Such fused cytochromes, consisting of P450 monooxygenase and P450 reductase, have been identified in both ascomycete and basidiomycete fungi, making the origin of this clan likely to predate the split of different fungal classes. Two single member families of the fungus were assigned to clan CYP54. The CYP families of this clan appear in filamentous ascomycete fungi and are likely involved in pathways leading to secondary metabolite synthesis (Deng 2006). Three *of R. oryzae* P450 families (14 CYPs) belonged to clan CYP64 and one single-membered orphan family might also be related to it. The original CYP64 gene was identified in ascomycete *Aspergillus flavus* as an enzyme associated with the synthesis of aflatoxin, a fungal secondary metabolite (Woloshuk et al. 1994).

### Example 12: Effect of Progesterone on the Expression Profiles of Selected P450s

The selection *of R. oryzae* putative P450 genes for expression studies was done based on data obtained by the fungus P450ome analysis. The genes of P450s assigned to clans CYP51, CYP61, CYP52, and CYP505 were excluded. The CYP5210 family of clan CYP56 displaying 25 - 29 % identity to P450s associated with the fumonisin biosynthetic pathway in *Fusarium species* (Seo et al. 2001; Proctor et al. 1999) was also omitted.
RT PCR was therefore performed for 31 putative P450 genes encoding CYPs of clans CYP64 (families CYP5206, CYP5207, CYP5208, CYP5209), CYP56 (families CYP509, CYP5212), and CYP54 (families CYP5204, CYP52013). The random-primed cDNA was synthesized using total RNA isolated from *R. oryzae* treated with progesterone for different time periods and from the fungus not exposed to steroids. The transcripts of two genes (CYP509C12 and CYP509C10) were detected only during exposure of the fungus to progesterone and seven genes were also expressed in control fungal mycelium under tested conditions (Figure 2). Further examination of expression profiles of these genes using real time RT PCR demonstrated that three of the genes (CYP5207A2, CYP5206A5, and CYP5206A4) appeared down-regulated during treatment of the fungus with progesterone (Figure 2). The transcript levels of these genes fell to less than one-third-fold in at least one time period during fungus' treatment in comparison to the control. CYP509B1 gene was initially down-regulated but showed up-regulation within 40 to 120 minutes of fungus' exposure to progesterone. The remaining five genes (CYP5208A1, CYP5213A1, CYP5204A1, CYP509C12, and CYP509C10) were up-regulated by progesterone within 20 minutes, albeit to different extent. The transcript amount of three of them (CYP5204A1, CYP509C12, CYP509C10) was increased (Wilcoxon's test, p value < 0.05) at least 32-fold compared to the control.
Two of the strongly inducible genes CYP509C12 and CYP509C10 were chosen for further investigation.

### Example 13: Alignment of Amino Acid Sequences of CYP509C12 and CYP509C10

Primary structure analysis of *R. oryzae* CYP509C12 (520 amino acids) and CYP509C10 (526 amino acids) showed high amino acid identity (58 %). The sequences included conserved eukaryotic P450 regions: a K-helix region with the conserved ExxR motif, an I helix region with the amino acid consensus sequence (A/G)Gx(D/E)T(T/S), a PERF domain in the aromatic region located 50 amino acids downstream of the K-helix, and the heme binding domain at the C-terminal end (PFGNGARQCxG) (Figure 3).

### Example 14: Expression of CYP509C12 and CYP509C10 in S. pombe

Full length cDNAs of CYP509C12 (1581 bp) and CYP509C10 (1587 bp) amplified from cDNA library *of R. oryzae* exposed to progesterone were cloned into pNMT1-TOPO expression vectors and transformed into *S*. *pombe,* producing yeast strains SpoC12 and SpoC10, respectively. After cultivation of the parental and recombinant *S. pombe* in the absence of thiamine, whole cell protein lysates were prepared. The expression of recombinant C-terminal hexahistidine tagged CYP509C12 and CYP509C10 was verified by Western blot analysis using polyclonal rabbit anti-His₆ antibodies. From the results presented in Figure 4 it can be seen that both *R. oryzae* enzymes were expressed and their molecular weight of approximately 60 kDa corresponded to their predicted molecular weight.

Due to the fact we did not detect biotransformation of progesterone with recombinant strain SpoC10, as stated below, the investigation of subcellular localization was done only for SpoC12.

Western blot analysis of subcellular fractions showed accumulation of the recombinant CYP509C12 protein in the microsomal fraction while it was not detected in the cytosolic fraction (Figure 4). The result suggests that the recombinantly expressed *R. oryzae* P450 is localized in the endoplasmic reticulum of the yeast.

### Abbreviations

The abbreviations used are: P450, cytochrome P450; RoCPR1, cytochrome P450 reductase from R. oryzae, DOC, 11-deoxycorticosterone; RSS, 11-deoxycortisol; Edinburgh minimal media, EMM; LC-MS, liquid chromatography - mass spectroscopy; HPLC, high performance liquid chromatography;

### References

1. Breskvar, K., and Hudnik-Plevnik, T. (1977) Biochem Biophys Res Commun 74, 1192-1198
2. Moreno, S., Klar, A., and Nurse, P. (1991) Methods Enzymol 194, 795-823
3. Alfa, C., Fantes, P., Hyams, J., and M., M. (1993) Experiments with Fission Yeast. A Laboratory Course Manual, Cold Spring Harbor Press, Cold Spring Harbor, NY
4. Kumar, S., Tamura, K., and Nei, M. (2004) Briefings in Bioinformatics 5, 150-163
5. Nelson, D. R. (1999) Arch Biochem Biophys 369, 1 - 10
6. Breskvar, K., Cresnar, B., Plaper, A., and Hudnik-Plevnik, T. (1991) Biochem Biophys Res Commun 178, 1078-1083
7. Ito, H., Fukuda, Y., Murata, K., Kimura, A. (1983) J Bacteriol. 153, 163-168
8. Andreadis, A., Hsu, Y. P., Hermodson, M., Kohlhaw, G., and Schimmel, P. (1984) J Biol Chem 259, 8059-8062
9. Oeda, K., Sakaki, T., and Ohkawa, H. (1985) DNA 4, 203-210
10. Bradford, M. M. (1976) Anal Biochem 72, 248-254
11. Laemmli, U. K. (1970) Nature 227, 680-&
12. Hudnik-Plevnik, T., and Cresnar, B. (1990) Journal of Steroid Biochemistry 35, 749-751
13. Sanglard, D., and Loper, J. C. (1989) Gene 76, 121 - 136
14. Briza, P., Kalchhauser, H., Pittenauer, E., Allmaier, G., and Breitenbach, M. (1996) European Journal of Biochemistry 239, 124-131
15. Kitazume, T., Takaya, N., Nakayama, N., and Shoun, H. (2000) J. Biol. Chem. 275, 39734-39740
16. Boddupalli, S. S., Pramanik, B. C., Slaughter, C. A., Estabrook, R. W., and Peterson, J. A. (1992) Archives of Biochemistry and Biophysics 292, 20-28
17. Deng, X. (2006) Structural, Functional and Evolutionary Analyses of the Rice Blast Fungal Genome. in Plant Pathology and Bioinformatics, North Carolina State University, Raleigh
18. Woloshuk, C. P., Foutz, K. R., Brewer, J. F., Bhatnagar, D., Cleveland, T. E., and Payne, G. A. (1994) Appl. Environ. Microbiol. 60, 2408-2414
19. Seo, J.-A., Proctor, R. H., and Plattner, R. D. (2001) Fungal Genetics and Biology 34, 155-165
20. Proctor, R. H., Desjardins, A. E., Plattner, R. D., and Hohn, T. M. (1999) Fungal Genetics and Biology 27, 100-112
21. Narusaka, M., Narusaka, Y., Seki, M., Ishida, J., Nakashima, M., Enju, A., Sakurai, T., Satou, M., and Shinozaki, K. (2004) Plant and Cell Physiology 45, S188-S188
22. Doddapaneni, H., and Yadav, J. S. (2005) Molecular Genetics and Genomics 274, 454-466
23. Makovec, T., and Breskvar, K. (2002) Journal of Steroid Biochemistry and Molecular Biology 82, 89-96
24. Yadav, J. S., and Doddapaneni, H. (2003) proceedings of the 13th Internat. Conf. On Cytochromes P450 Biochemistry, Biophysics And Drug Metabolism: June 29-July 3, Prague (Czech Republic), 333 - 340
25. Yadav, J. S., Doddapaneni, H., and Subramanian, V. (2006) Biochemical Society Transactions 34, 1165-1169
26. Kunic, B., Truan, G., Breskvar, K., and Pompon, D. (2001) Molecular Genetics and Genomics 265, 930-940
27. Hannemann, F., Bichet, A., Ewen, K. M., and Bernhardt, R. (2007) Biochimica Et Biophysica Acta-General Subjects 1770, 330-344
28. Hakki, T., Zearo, S., Dragan, C. A., Bureik, M., and Bernhardt, R. (2008) J Biotechnol 133, 351-359

## Claims

1. Use of a recombinant protein having the sequence set forth in SEQ ID NO:1, or a protein having a sequence which is at least 50% identical to SEQ ID NO:1 and has the same catalytic function as the protein set forth in SEQ ID NO:1, for the biotransformation of a steroid.

2. Use as defined in claim 1, wherein said steroid is progesterone, testosterone, 11-deoxycorticosterone, and 11-deoxycortisol.

3. Use of a gene coding for a recombinant protein having the sequence set forth in SEQ ID NO:1, or a gene coding for a protein having a sequence which is at least 50% identical to SEQ ID NO:1 and having the same catalytic function as the protein of SEQ ID NO:1, for the production of a recombinant cell capable of biotransformation of steroids.

4. Use as defined in claim 3, wherein said recombinant cell is a *Schizosaccaromyces* cell.

5. A recombinant cell comprising a recombinant protein having the sequence set forth in SEQ ID NO:1, or a protein having a sequence which is at least 50% identical to SEQ ID NO:1 and has the same catalytic function as the protein set forth in SEQ ID NO:1, said recombinant cell being capable of biotransformation of a steroid.

6. The recombinant cell of claim 5, wherein said cell is capable of biotransformation of progesterone, testosterone, 11-deoxycorticosterone, and 11-deoxycortisol.

7. The recombinant cell of claim 5 or 6, wherein said recombinant cell is *a Schizosaccaromyces* cell.

8. The recombinant cell of any one of claims 5 to 7, wherein said cell further comprises a recombinant cytochrome P450 reductase.

9. Use of the recombinant cell of any one of claims 5 to 8 for the biotransformation of a steroid.

10. Use of claim 9, wherein said biotransformation of a steroid is hydroxylation of progesterone, testosterone, 11-deoxycorticosterone, and 11-deoxycortisol.

11. Method for the production of a product steroid from a substrate steroid, said method comprising (a) incubating said substrate steroid with a recombinant cell of any one of claims 5 to 8 in a suitable medium, whereby said substrate steroid is converted to said product steroid, and (b) obtaining said product steroid from the medium.

12. Method of claim 11, wherein said substrate steroid is selected from the group consisting of progesterone, testosterone, 11-deoxycorticosterone, and 11-deoxycortisol.

13. Method of claim 11 or 12, wherein said substrate steroid is hydroxylated.

14. Method of any of claims 11 to 13, wherein the product steroid is 11α-progesterone.

## Patentansprüche

1. Verwendung eines rekombinanten Proteins, das die in SEQ ID NO: 1 ausgeführte Sequenz aufweist, oder eines Proteins, das eine Sequenz aufweist, die mindestens 50 % mit SEQ ID NO: 1 identisch ist und dieselbe katalytische Funktion wie das in SEQ ID NO: 1 aufgeführte Protein aufweist, für die Biotransformation eines Steroids.

2. Verwendung wie in Anspruch 1 definiert, wobei es sich bei dem Steroid um Progesteron, Testosteron, 11-Desoxycorticosteron und 11-Desoxycortison handelt.

3. Verwendung eines Gens, das ein rekombinantes Protein kodiert, welches die in SEQ NO: 1 ausgeführte Sequenz aufweist, oder eines Gens, das ein Protein kodiert, welches eine Sequenz aufweist, die mindestens 50 % mit SEQ ID NO:1 identisch ist und dieselbe katalytische Funktion wie das Protein von SEQ ID NO: 1 aufweist, für die Herstellung einer rekombinanten Zelle, die zur Biotransformation von Steroiden in der Lage ist.

4. Verwendung wie in Anspruch 3 definiert, wobei es sich bei der rekombinanten Zelle um eine *Schizosaccharomyces-Zelle* handelt.

5. Rekombinante Zelle, welche ein rekombinantes Protein aufweist, das die in SEQ ID NO: 1 ausgeführte Sequenz aufweist, oder ein Protein, welches eine Sequenz aufweist, die mindestens 50 % mit SEQ ID NO: 1 identisch ist und dieselbe katalytische Funktion wie das in SEQ ID NO: 1 ausgeführte Protein aufweist, wobei die rekombinante Zelle zur Biotransformation eines Steroids in der Lage ist.

6. Rekombinante Zelle nach Anspruch 5, wobei die Zelle zur Biotransformation von Progesteron, Testosteron, 11-Desoxycorticosteron und 11-Desoxycortison in der Lage ist.

7. Rekombinante Zelle nach Anspruch 5 oder 6, wobei es sich bei der rekombinanten Zelle um eine *Schizosaccharomyces*-Zelle handelt.

8. Rekombinante Zelle nach einem der Ansprüche 5 bis 7, wobei die Zelle des Weiteren eine rekombinante Cytochrom-P450-Reduktase aufweist.

9. Verwendung der rekombinanten Zelle nach einem der Ansprüche 5 bis 8 für die Biotransformation eines Steroids.

10. Verwendung nach Anspruch 9, wobei es sich bei der Biotransformation eines Steroids um die Hydroxylierung von Progesteron, Testosteron, 11-Desoxycorticosteron und 11-Desoxycortison handelt.

11. Verfahren für das Herstellen eines Steroidproduktes aus einem Steroidsubstrat, wobei das Verfahren (a) das Inkubieren des Steroidsubstrates mit einer rekombinanten Zelle nach einem der Ansprüche 5 bis 8 in einem geeigneten Medium, wobei das Steroidsubstrat zu dem Steroidprodukt umgewandelt wird, und (b) das Erhalten des Steroidproduktes aus dem Medium umfasst.

12. Verfahren nach Anspruch 11, wobei das Steroidsubstrat aus der Gruppe ausgewählt ist, die aus Progesteron, Testosteron, 11-Desoxycorticosteron und 11-Desoxycortison besteht.

13. Verfahren nach Anspruch 11 oder 12, wobei das Steroidsubstrat hydroxyliert ist.

14. Verfahren nach einem der Ansprüche 11 bis 13, wobei es sich bei dem Steroidprodukt um 11α-Progesteron handelt.

## Revendications

1. Utilisation d'une protéine recombinante ayant la séquence exposée dans SEQ ID NO : 1, ou d'une protéine ayant une séquence qui présente une identité d'au moins 50 % avec SEQ ID NO : 1 et qui a la même fonction catalytique que la protéine exposée dans SEQ ID NO : 1, pour la biotransformation d'un stéroïde.

2. Utilisation selon la revendication 1, dans laquelle ledit stéroïde est la progestérone, la testostérone, la 11-désoxycorticostérone, et le 11-désoxycortisol.

3. Utilisation d'un gène codant pour une protéine recombinante ayant la séquence exposée dans SEQ ID NO : 1, ou d'un gène codant pour une protéine ayant une séquence qui présente une identité d'au moins 50 % avec SEQ ID NO : 1 et ayant la même fonction catalytique que la protéine de SEQ ID NO : 1, pour la production d'une cellule recombinante capable de biotransformation de stéroïdes.

4. Utilisation selon la revendication 3, dans laquelle ladite cellule recombinante est une cellule de *Schizosaccharomyces.*

5. Cellule recombinante comprenant une protéine recombinante ayant la séquence exposée dans SEQ ID NO : 1, ou d'une protéine ayant une séquence qui présente une identité d'au moins 50 % avec SEQ ID NO : 1 et qui a la même fonction catalytique que la protéine exposée dans SEQ ID NO : 1, ladite cellule recombinante étant capable de biotransformation d'un stéroïde.

6. Cellule recombinante selon la revendication 5, ladite cellule étant capable de biotransformation de la progestérone, de la testostérone, de la 11-désoxycorticostérone, et du 11-désoxycortisol.

7. Cellule recombinante selon la revendication 5 ou 6, ladite cellule recombinante étant une cellule de *Schizosaccharomyces.*

8. Cellule recombinante selon l'une quelconque des revendications 5 à 7, ladite cellule comprenant en outre une réductase recombinante du cytochrome P450.

9. Utilisation de la cellule recombinante selon l'une quelconque des revendications 5 à 8, pour la biotransformation d'un stéroïde.

10. Utilisation selon la revendication 9, dans laquelle ladite biotransformation d'un stéroïde est l'hydroxylation de la progestérone, de la testostérone, de la 11-désoxycorticostérone, et du 11-désoxycortisol.

11. Procédé de production d'un produit stéroïde à partir d'un substrat stéroïde, ledit procédé comprenant (a) l'incubation dudit substrat stéroïde avec une cellule recombinante selon l'une quelconque des revendications 5 à 8 dans un milieu adapté, ledit substrat étant ainsi converti en ledit produit stéroïde, et (b) l'obtention dudit produit stéroïde du milieu.

12. Procédé selon la revendication 11, dans lequel ledit substrat stéroïde est choisi dans le groupe constitué par la progestérone, la testostérone, la 11-désoxycorticostérone, et le 11-désoxycortisol.

13. Procédé selon la revendication 11 ou 12, dans lequel ledit substrat stéroïde est hydroxylé.

14. Procédé selon l'une quelconque des revendications 11 à 13, dans lequel le produit stéroïde est la 11α-progestérone.


### SEQUENCE LISTING

<110> Univerza v Ljubljani
<120> Cytochrome P450 from Rhizopus oryzae and uses thereof <130> P003113ep2

   <160> 3
   <170> PatentIn version 3.3
   <210> 1
   <211> 526
   <212> PRT
   <213> Rhizopus oryzae
   <400> 1
   Met Met Glu Ile Ala Glu Phe Ala Leu Glu Ser Tyr Arg Ser Thr Leu
   1               5                   10                  15
   Glu Lys Ile Leu Pro Val Leu Gln Lys Lys Ser Lys Lys Ser Tyr Ile
               20                  25                  30
   Gly Ala Ala Ile Thr Leu Ile Val Leu His Arg Ile Tyr Ser Tyr Phe
           35                  40                  45
   Lys Val Pro Lys Arg Phe Gln His Leu Pro Lys Leu Ser Tyr Phe Pro
       50                  55                  60
   Ser Ala Lys Ser Ile Phe Asn Asn Glu Pro Ile Tyr Asp Arg Tyr Lys
   65                  70                  75                  80
   Arg Leu Val Phe Pro Val Ile Lys Glu Asn Asn Gly Ile Tyr Val Ser
                   85                  90                  95
   Lys Ile Ser Phe Asp Trp Thr Val Tyr Ile Ala Asn Pro Val Ala Ala
               100                 105                 110
   Lys His Val Leu Leu Lys Ala Asp Leu Tyr Pro Lys Ser His Asp Phe
           115                 120                 125
   Leu Lys Met Leu Gly Ser Asn Ser Pro Val Val Gln Phe Leu Gly Tyr
       130                 135                 140
   Asp Ser Val Gly Leu Thr Asn Gly His Val Trp Lys Asn Gln Arg Lys
   145                 150                 155                 160
   Leu Met Asn Pro Ala Phe His Arg Ser Met Pro Ile Asn Thr Met Ser
                   165                 170                 175
   Thr Val Ile Pro Asp Leu Phe Phe Val Ile Glu Lys Glu Asn Gly Thr
               180                 185                 190
   Ile Ala Val Pro Ser Val Met Arg Asp Phe Thr Leu Asp Val Leu Gly
           195                 200                 205
   Leu Thr Val Phe Gly Phe Asp Phe Lys Ala Leu Lys Gly Asp Pro Asp
       210                 215                 220
   Glu Trp Thr Lys Thr Phe Thr Leu Ala Asn Glu Gly Leu Phe Asp Pro
   225                 230                 235                 240
   Ile Leu Asn Ile Phe Gly Pro Phe Ser Phe Ile Leu Thr Ala Ile Phe
                   245                 250                 255
   Pro Lys Arg Arg Glu Gln Leu Lys Ala Val Ala Lys Leu Asn Gly Lys
               260                 265                 270
   Leu Glu Gln Leu Ile His Gln Lys Arg Met Glu Ile Glu Asn Gly Ala
           275                 280                 285
   Tyr Ser Asn Thr Pro Glu Asn Glu Lys Asp Leu Val Ala Leu Met Leu
       290                 295                 300
   Glu Ala Glu Lys Arg Gly Glu Gly Leu Thr Asn Asp Leu Glu Leu Arg
   305                 310                 315                 320
   His Asn Ile Ala Gly Phe Phe Leu Ala Gly His Asp Thr Thr Ala Asn
                   325                 330                 335
   Ala Leu Ser Phe Cys Phe Tyr Asn Leu Ala Lys Asn Lys His Val Gln
               340                 345                 350
   Asn Lys Leu Arg Gln Glu Ile Ile Ser Val Leu Gly Asp Asp Pro Lys
           355                 360                 365
   Asp Val Val Pro Thr Leu Asp Gln Leu Lys Glu Met Pro Tyr Leu Asn
       370                 375                 380
   Leu Val Leu Lys Glu Asn Leu Arg Leu Asn Gly Pro Ala Asp Asn Ile
   385                 390                 395                 400
   Leu Pro Arg Val Ala Ala Lys Asp Met Val Val Asp Gly Thr Phe Ile
                   405                 410                 415
   Pro Lys Gly Ala Thr Val Asn Ile Asp Ile Tyr Gly Ile His His Asn
               420                 425                 430
   Pro Lys Phe Trp Asn Asn Pro Asp Asp Phe Ile Pro Glu Arg Leu Asp
           435                 440                 445
   Glu Asn Gly Glu Gln Asp Ser His Asp Gly Leu Thr Trp Leu Pro Phe
       450                 455                 460
   Gly Asn Gly Ala Arg Gln Cys Leu Gly Met Asn Phe Ser Leu Thr Glu
   465                 470                 475                 480
   Gln Arg Leu Leu Leu Val Met Met Ile Arg Lys Tyr Glu Ile Asp Val
                   485                 490                 495
   Pro Lys Asp Ser Ile His Tyr Glu Arg Val Ile Phe Gly Ser Glu Thr
               500                 505                 510
   Thr Pro Pro Asn Ser Leu Glu Leu Thr Phe Lys Lys Arg Tyr
           515                 520                 525
<210> 2
   <211> 526
   <212> PRT
   <213> Rhizopus oryzae
   <400> 2
   Met Glu Leu Val Gln Phe Val Asp Lys Ser Tyr Arg Glu Ile Leu Glu
   1 5 10 15
   Lys Leu Leu Pro Val Leu Gln Lys Lys Ser Lys Trp Ser Tyr Ile Ser
               20                  25                  30
   Ala Ala Ala Leu Leu Ile Thr Ile Gln Gln Ile Tyr Ser Tyr Leu His
           35                  40                  45
   Val Pro Lys Lys Phe Arg His Ile Pro Ser Val Ser Ser Leu Ala Met
       50                  55                  60
   Ala Met Ser Phe Leu Arg His Glu Ser Gln Ala Asp Arg Phe Lys Arg
   65                  70                  75                  80
   Ile Ile Gln Pro Val Met Asn Lys Gly Asn Gly Val Tyr Val Ser Lys
                   85                  90                  95
   Ile Pro Ile Ser Trp Thr Val Tyr Val Ala Asn Pro Val Ile Ala Lys
               100                 105                 110
   His Ile Leu Leu Lys Ser Asp Met Tyr Pro Lys Ser His Asn Gly Phe
           115                 120                 125
   Lys Ile Ile Gly Pro Asn Ser Pro Phe Leu Gln Phe Ile Gly Leu Asn
       130                 135                 140
   Asn Leu Ala Leu Ser Asn Gly His Thr Trp Lys Lys Gln Arg Lys Leu
   145                 150                 155                 160
   Met Asn Pro Val Phe His Arg Ser Met Pro Val Lys Met Met Ser Ser
                   165                 170                 175
   Val Leu Leu Thr Leu Phe Ser Ala Ile Glu Lys Ala Asp Gly Thr Ile
               180                 185                 190
   Pro Ile Ala Glu Ala Met Lys His Phe Thr Leu Asp Val Leu Gly Tyr
           195                 200                 205
   Thr Ile Phe Asp Phe Asp Phe Lys Ala Leu Lys Gly Asp Pro Asp Asp
       210                 215                 220
   Trp Thr Lys Thr Tyr His Leu Val Asn Glu Ala Leu Ala Asp Pro Val
   225                 230                 235                 240
   Leu Asn Val Phe Pro Ser Met Gly Ala Leu Leu Leu Val Leu Leu Pro
                   245                 250                 255
   Val Lys Arg Arg Arg Met Ala Ala Ile Asp Lys Leu Asn Arg Lys Leu
               260                 265                 270
   Asp Glu Met Ala Gln Lys Lys Arg Arg Glu Ile Gln Lys Gly Ser Tyr
           275                 280                 285
   Ser Asn Lys Leu Asp Ser Glu Lys Asp Leu Leu Thr Leu Met Leu Glu
       290                 295                 300
   Ala Glu Asn Asn Gly Glu Gly Leu Leu Ser Asp Thr Glu Leu Arg His
   305                 310                 315                 320
   Asn Leu Ala Thr Phe Phe Leu Ala Gly His Glu Thr Thr Ala Asn Ser
                   325                 330                 335
   Leu Ser Phe Ala Phe Tyr Tyr Leu Ala Gln Asn Lys His Val Gln Gln
           340                 345                 350
   Lys Leu Arg Glu Glu Val Ile Ser Ile Phe Gly Asp Glu Pro Thr Asp
       355                 360                 365
   Ile Ala Pro Thr Leu Glu Gln Leu Lys Gln Leu Ser Tyr Leu Asp Leu
       370                 375                 380
   Val Ile Lys Glu Thr Leu Arg Ile Ala Gly Pro Ile Asp Arg Ile Val
   385                 390                 395                 400
   Pro Arg Val Val Ser Glu Asp Ile Val Val Gly Gly Ala Phe Ile Pro
                   405                 410                 415
   Lys Gly Thr Ser Ala Asn Ile Asp Leu Tyr Ala Ile His His Asn Thr
               420                 425                 430
   Asn Ile Trp Lys Asn Pro Asp Gln Phe Ile Pro Glu Arg Phe Ala Glu
           435                 440                 445
   Gly Gly Glu Gln Glu Ser His Glu Gly Leu Thr Trp Val Pro Phe Gly
       450                 455                 460
   Asn Gly Ala Arg Gln Cys Ile Gly Met Asn Phe Ser Leu Ala Glu Gln
   465                 470                 475                 480
   Arg Ile Val Leu Ala Leu Met Val Arg Lys Tyr Ile Ile Asp Ile Pro
                   485                 490                 495
   Lys Asp Ser Ile His Tyr Asp His Val Val Phe Asp Ser Thr Glu Thr
               500                 505                 510
   Lys Ala Pro Glu Ser Leu Lys Leu Lys Phe Thr Lys Arg His
           515                 520                 525
<210> 3
   <211> 713
   <212> PRT
   <213> Rhizopus oryzae
   <400> 3
   Met Thr Arg Asn Asn Ser His His Leu Leu Asp Thr Val Asp Leu Ile
   1               5                   10                  15
   Leu Leu Gly Thr Ile Gly Leu Gly Thr Val Ala Trp Phe Ala Arg His
               20                  25                  30
   Gln Ile Ala Asn Arg Leu Phe Lys Ser Asp Ser Thr Asn Lys Ser Glu
           35                  40                  45
   Val Lys Asp Glu Ala Lys Thr Pro Lys Gln Glu Arg Asn Phe Val Lys
       50                  55                  60
   Val Met Gln Gln Gln Gly Arg Arg Val Ile Phe Phe Tyr Gly Ser Gln
   65                  70                  75                  80
   Thr Gly Thr Ala Glu Asp Phe Ala Ser Arg Leu Ala Lys Glu Cys Thr
                   85                  90                  95
   Gln Lys Tyr Gly Val Ser Ala Met Thr Ala Asp Ile Glu Gln Tyr Asp
               100                 105                 110
   Leu Ser Tyr Leu Asp Ser Val Pro Glu Asp Ser Leu Val Phe Phe Ile
           115                 120                 125
   Met Ala Thr Tyr Gly Glu Gly Glu Pro Thr Asp Asn Ala Val Asp Phe
       130                 135                 140
   Trp Asp Leu Leu Ala Glu Glu Val Pro Glu Phe Ser Asn Asp Asp Gly
   145                 150                 155                 160
   Glu Gly Lys Pro Leu Gln Lys Leu Arg Tyr Val Ala Phe Gly Leu Gly
                   165                 170                 175
   Asn Lys Thr Tyr Glu His Tyr Asn Glu Val Ile Arg Lys Val Asp Asn
               180                 185                 190
   Arg Leu Leu Ser Leu Gly Ala Lys Arg Ile Gly Glu Arg Gly Glu Gly
           195                 200                 205
   Asp Asp Asp Gly Thr Leu Glu Glu Asp Phe Leu Ala Trp Gln Glu Glu
       210                 215                 220
   Met Trp Pro Ala Phe Cys Glu Ala Leu Gly Val Asp Glu Ser Asn Ala
   225                 230                 235                 240
   His Ser Gly Pro Arg Gln Ala Ile Phe Lys Ile Glu Glu Leu Thr Ala
                   245                 250                 255
   Tyr Asp Gln Ala Lys Val Tyr Leu Gly Glu Ile Gly Glu Trp Leu Lys
               260                 265                 270
   Glu Gly Ala Ser Ile Val Tyr Asp Ala Lys Arg Pro Tyr Asn Ala Pro
           275                 280                 285
   Ile Thr Ser Lys Asp Ile Phe Lys Ala Gly Asp Arg His Cys Leu His
       290                 295                 300
   Leu Glu Ile Asp Ile Ser Asn Thr Asn Leu Ser Tyr Gln Thr Gly Asp
   305                 310                 315                 320
   His Val Ala Ile Trp Pro Thr Asn Asn Glu Val Glu Val Asn Arg Leu
                   325                 330                 335
   Ala Lys Leu Leu Gly Leu Gln Asn Lys Leu Asp Thr Val Ile His Val
               340                 345                 350
   Gln Ser Leu Asp Pro Ala Ala Ser Lys Lys Tyr Pro Phe Pro Val Pro
           355                 360                 365
   Thr Thr Tyr Arg Ala Val Phe Arg His Tyr Leu Asp Ile Cys Ser Ala
       370                 375                 380
   Val Pro Arg Gln Val Leu Met Ser Leu Ile Glu Tyr Ala Pro Thr Glu
   385                 390                 395                 400
   Ala Ser Lys Glu Ala Leu Arg Lys Leu Ala Thr Asp Lys Asp Glu Tyr
                   405                 410                 415
   Arg Val His Val Gly Asp Val Thr Arg Asn Leu Gly Glu Val Leu Gln
               420                 425                 430
   Met Leu Ala Glu Ser Glu Ser Leu Glu Leu Asp Gly Ala Phe Ser Ser
               435                 440                 445
   Val Pro Phe Asp Leu Val Ile Glu Ser Ile Ser Arg Leu Gln Pro Arg
       450                 455                 460
   Tyr Tyr Ser Ile Ser Ser Ser Ser Lys Glu Asn Pro Lys Lys Ile Ala
   465                 470                 475                 480
   Val Thr Ala Val Thr Leu Gln Tyr Thr Pro Glu His Gly Ser Pro Arg
                   485                 490                 495
   Thr Val Tyr Gly Val Asn Thr Asn Tyr Leu Trp Arg Val His Glu Ala
               500                 505                 510
   Val Asn Asn Leu Thr Pro Asn Ser Val Ile Pro Glu Tyr Asn Leu Thr
           515                 520                 525
   Gly Pro Arg Asp Ser Leu Phe Ser Gln Gln Gly Lys Val Ala Arg Ile
       530                 535                 540
   Pro Val His Val Arg Arg Ser Gln Phe Lys Leu Pro Arg Asn Pro Thr
   545                 550                 555                 560
   Val Pro Val Ile Met Ile Gly Pro Gly Thr Gly Val Ala Pro Phe Arg
                   565                 570                 575
   Gly Phe Val Arg Glu Arg Ala Leu Gln Lys Lys Glu Asn Lys Pro Val
               580                 585                 590
   Gly Pro Thr Ile Leu Phe Phe Gly Cys Arg Asn Arg Ala Glu Asp Phe
           595                 600                 605
   Leu Tyr Glu Glu Glu Trp Pro Glu Leu Phe Glu Val Leu Gly Gly Asp
       610                 615                 620
   Ser Arg Ile Ile Thr Ala Phe Ser Arg Glu Thr Glu Lys Lys Val Tyr
   625                 630                 635                 640
   Val Gln His Arg Leu Met Glu Asn Gly Gln Glu Met Trp Asn Leu Leu
                   645                 650                 655
   Glu Lys Gly Ala Tyr Val Tyr Val Cys Gly Asp Ala Lys Asn Met Ala
               660                 665                 670
   Arg Asp Val Asn Gln Thr Phe Val Arg Phe Ala Gln Gln Phe Gly Gly
           675                 680                 685
   Met Asp Glu Asn Arg Ser Gln Asp Tyr Val Lys Asn Leu Arg Asn Thr
       690                 695                 700
   Gly Arg Tyr Gln Glu Asp Val Trp Ser
   705                 710